# EUROPÄISCHE PATENTSCHRIFT

(11) **EP 2 686 655 B1**
(45) Veröffentlichungstag und Bekanntmachung des Hinweises auf die Patenterteilung: **24.08.2016**
(21) Anmeldenummer: 12735089.0
(22) Anmeldetag: 26.06.2012
(51) Int. Cl.: G01M 3/10

(54) **PRÜFVERFAHREN UND -VORRICHTUNG FÜR BIOREAKTORBEHÄLTER SOWIE VERWENDUNG**
TEST METHOD AND APPARATUS FOR BIOREACTOR CONTAINERS AND USE
PROCÉDÉ ET DISPOSITIF DE CONTRÔLE DE RÉSERVOIRS DE BIORÉACTEUR ET UTILISATION ASSOCIÉE

(30) Priorität: 30.06.2011 DE 102011106165
(43) Veröffentlichungstag der Anmeldung: 22.01.2014
(60) Teilanmeldung: 15001756.4
(73) Patentinhaber: Sartorius Stedim Biotech GmbH, 37079 Göttingen (DE)
(72) Erfinder: DAHLBERG, Martin, 37120 Bovenden (DE); GAY, Isabelle, F-13124 Peypin (FR); BÖTTCHER, Lars, 34212 Melsungen (DE); OBERMANN, Stefan, 37139 Adelebsen (DE); SANDROCK, Rainer, 34134 Kassel (DE)
(74) Vertreter: Müller-Boré & Partner Patentanwälte PartG mbB
(86) Internationale Anmeldenummer: PCT/EP2012/002686
(87) Internationale Veröffentlichungsnummer: WO 2013/000565

(56) Entgegenhaltungen:
- EP-A1- 0 060 548
- EP-A1- 0 209 203
- EP-A1- 1 283 061
- WO-A1-95/00827
- WO-A1-03/023351
- WO-A1-2009/093995
- WO-A1-2011/064067
- DE-A1-102007 057 944
- DE-T2- 69 509 833
- US-A- 4 805 445

## Beschreibung

Die vorliegende Beschreibung betrifft ein Verfahren und eine Vorrichtung zum zerstörungsfreien Prüfen der Integrität von Bioreaktorbehältern sowie eine Verwendung einer fluiddurchlässigen und/oder strukturierten Aufnahmeschicht.

In der pharmazeutischen und biotechnologischen Industrie werden flexible Behälter, wie zum Beispiel Beutel, als Bioreaktorbehälter für die Prozeßführung bzw. Lagerung verwendet. Die Bioreaktorbehälter können vor der eigentlichen Verwendung durch den Herstellungsprozeß, Transport oder Handhabung beschädigt werden. Es empfiehlt sich daher, vor der eigentlichen Verwendung einen Integritätstest des Bioreaktorbehälters durchzuführen. Ein Integritätstest ist auch nach der Verwendung des Bioreaktorbehälters sinnvoll, um nachzuweisen, daß während der gesamten Prozeßführung die Integrität des Bioreaktorbehälters aufrecht erhalten wurde. Der Begriff "Bioreaktorbehälter" im Sinne der Anmeldung umfaßt gleichermaßen Bioreaktoren sowie Behälter mit bereichsweise flexiblen Wänden, die beispielsweise zur Aufnahme, zum Mischen, zur Speicherung und zum Spenden von sterilen Medien dienen.

Gängige Testmethoden zur Integritätsprüfung des Bioreaktorbehälters sind die Druckabfallmethode, die Durchflußmessung und Spurengasanalyse unter Verwendung eines Testgases. Allen Testmethoden gemein ist, daß zwischen dem Innenraum des Bioreaktorbehälters und einer den Bioreaktorbehälter aufnehmenden Testvorrichtung bzw. der Umgebung des Bioreaktorbehälters ein Differenzdruck erzeugt wird. Dazu wird nach dem Herstellen bzw. vor und/oder nach der Benutzung der zu prüfende Bioreaktorbehälter in eine Testvorrichtung zum Prüfen der Integrität angeordnet. Dabei besteht die Gefahr, daß der zu prüfende Bioreaktorbehälter bei der dazu notwendigen Handhabung beschädigt wird, so daß in dem an sich dichten Bioreaktorbehälter ein Leck entsteht.

Die Druckschrift EP 0 060 548 A1 offenbart ein Verfahren zur Dichtheitsprüfung elektrischer, einen Innenhohlraum in einem geschlossenen Gehäuse enthaltender Bauelemente sowei eine Vorrichtung zur Durchführung des Verfahrens. Die EP 0 209 203 A1 offenbart eine Einheit zur Detektion von Lecks mittels eines flexiblen Beutels.

Die Druckschrift WO 2009/093 995 A1 offenbart ein System zum Aufbewahren und Verarbeiten von Fluiden, insbesondere mit faltbaren Beuteln, wobei das System eine Vorrichtung zum Entfernen von Beutelfalten sowie eine Vorrichtung zur Detektion von Lecks aufweist.

Die Druckschrift US 4 805 445 offenbart ein Lagervorrichtung für ein unter Druck stehendes flüssiges Produkt in einem dichten Innenbehälter, der von einem steifen und dichten Außenbehälter umgebenen ist, wobei zwischen dem Innenbehälter und dem Außenbehälter ein Zwischenraum vorgesehen ist. Der Außenbehälter liegt an zementierten oder betonierten Wänden einer unterirdischen Aushöhlung an.

Es ist daher eine Aufgabe der Erfindung ein Verfahren und ein Vorrichtung zum Prüfen der Integrität von Bioreaktorbehältern bereitzustellen, welche(s) eine verbesserte Integritätsprüfung des Bioreaktorbehälters erlaubt.

Die Aufgabe wird durch die Merkmale der unabhängigen Ansprüche gelöst. Bevorzugte Ausführungsformen sind Gegenstand der abhängigen Ansprüche.

### Verfahren zum Prüfen der Integrität eines Bioreaktorbehälters

Ein Aspekt betrifft ein Verfahren zum Prüfen der Integrität eines Bioreaktorbehälters mit den Schritten:
- Bereitstellen eines Bioreaktorbehälters mit einer fluiddichten, zumindest bereichsweise flexiblen Wandung und mit zumindest einer Behälteröffnung;
- Bereitstellen einer Prüfvorrichtung mit einer Bioreaktorbehälteraufnahme und einer fluiddurchlässigen und/oder strukturierten Aufnahmeschicht, welche austauschbar an der Bioreaktorbehälteraufnahme angeordnet ist;
- Anordnen des Bioreaktorbehälters zumindest teilweise in der Bioreaktorbehälteraufnahme, wobei der Bioreaktorbehälter mit seiner Wandung zumindest bereichsweise die Aufnahmeschicht in der Bioreaktorbehälteraufnahme kontaktiert;
- Verbinden der zumindest einen Behälteröffnung des Bioreaktorbehälters mit einer Fluidquelle;
- Füllen des Bioreaktorbehälters mit einem Fluid aus der Fluidquelle, um zu einem ersten Zeitpunkt T₁ einen vorbestimmten Überdruck P₁ in dem Bioreaktorbehälter zu erzeugen;
- Diskriminieren, ob der Bioreaktorbehälter hinreichend dicht ist.

Dabei kann das Diskriminieren insbesondere anhand einer Druckdifferenz (P₂-P₁) zwischen einem Überdruck P₂ zu einem späteren Zeitpunkt T₂ und dem bestimmten Überdruck zu Zeitpunkt T₁ erfolgen. Alternativ oder zusätzlich kann das Diskriminieren anhand einer Fluidmenge M erfolgen, welche nach dem Zeitpunkt T₁ dem Bioreaktorbehälter zugeführt wurde, um den Überdruck P₁ konstant zu halten. Weiter alternativ oder zusätzlich kann das Diskriminieren anhand einer Detektion von Fluidstoffen bzw. Fluidpartikeln außerhalb oder innerhalb des Bioreaktorbehälters erfolgen, welche dem Bioreaktorbehälter zugeführt wurden. Vorteilhafterweise kann die Genauigkeit des Diskriminierens bzw. des Bestimmens und Entscheidens, ob der Bioreaktorbehälter dicht ist, erhöht werden, wenn zwei oder drei der oben beschriebenen Größen erfaßt werden. Die Wahrscheinlichkeit, daß die Dichtigkeit des Bioreaktorbehälters falsch klassifiziert bzw. bestimmt wird, sinkt dadurch vorteilhafterweise.

Ein Integritätstest gibt Auskunft über die Integrität bzw. Dichtigkeit der Bioreaktorbehälters, insbesondere ob Fluide aus einem Leck austreten oder eindringen können. Insbesondere ist die Integrität verletzt und der Bioreaktorbehälter unbrauchbar, wenn Mikroorganismen durch ein Leck in das Innere des Bioreaktorbehälters eindringen können, wodurch die Reaktion innerhalb des Bioreaktorbehälters beeinträchtigt und die entstandenen Produkte unbrauchbar sind.

Ein Bioreaktorbehälter umfaßt zumindest eine fluiddichte Wandung, welche zumindest bereichsweise flexibel ist. Der Begriff "Bioreaktorbehälter" im Sinne der Erfindung umfaßt gleichermaßen Bioreaktoren sowie Behälter mit bereichsweise flexiblen Wänden, die beispielsweise zur Aufnahme, zum Mischen, zur Speicherung und zum Spenden von sterilen Medien dienen. Dadurch ist der Bioreaktorbehälter volumenvariabel. Mit anderen Worten kann sich das Innenvolumen des Bioreaktorbehälters mit dem Befüllen vergrößern und mit dem Entleeren verringern. Weiterhin kann sich der Bioreaktorbehälter mit der flexiblen Wandung zumindest bereichsweise an die Bioreaktorbehälteraufnahme bzw. an die darin bzw. daran angeordnete Aufnahmeschicht anschmiegen. Zum Befüllen und Entleeren mit einem Fluid weist der Bioreaktorbehälter zumindest eine Behälteröffnung auf. Der Bioreaktorbehälter kann ein steriler Behälter bzw. steriler Bioreaktorbehälter sein.

Der Begriff "flexibel" umfaßt im Sinne der Anmeldung eine plastische sowie eine elastische Verformbarkeit. Der Begriff "Fluid" umfaßt sowohl eine gasförmige Phase, eine flüssige Phase als auch ein Gemisch aus flüssiger und gasförmiger Phase eines Stoffes.

Die Wandung des Bioreaktorbehälters besteht aus einem fluiddichten Material, welches im Hinblick auf die durchzuführende Reaktion biologisch bzw. chemisch inert ist, das heißt, daß die Wandung selbst im wesentlichen während der Reaktion nicht im biologischen bzw. chemischen Sinne reagiert. Bevorzugt umfaßt die Wandung ein Polymer, wie beispielsweise Polyethylen (PE) und/oder Polypropylen (PP). Weiter bevorzugt ist der Bioreaktorbehälter sterilisierbar, beispielsweise mittels Heißdampf, Plasmabehandlung, Ethylenoxidbegasung oder Gammabestrahlung, um die Reaktion unter sterilen Bedingungen beginnen zu können. Weiter bevorzugt sind die Behälteröffnungen als Sterilverbinder ausgebildet oder mit Sterilverbindern fluidisch verbunden, beispielsweise über einen Schlauch.

Die Wandung des Bioreaktorbehälters ist zumindest bereichsweise flexibel ausgebildet. Es versteht sich, daß die Wandung bereichsweise starr ausgebildet sein kann. Insbesondere im Bereich der zumindest einen Behälteröffnung kann die Wandung im wesentlichen starr ausgebildet sein, damit die zumindest eine Behälteröffnung formstabil ist.

Bioreaktorbehälter zur Durchführung einer biologischen Reaktion können bevorzugt ein Innenvolumen von etwa 5 Milliliter bis etwa 3000 Liter, bevorzugt etwa 2 Liter, etwa 5 Liter, etwa 10 Liter, etwa 50 Liter, etwa 100 Liter, etwa 250 Liter, etwa 500 Liter oder etwa 1000 Liter aufweisen. Üblicherweise sind Bioreaktorbehälter bei betriebsgemäßem Gebrauch mit einer wässrigen Lösung gefüllt, so daß dementsprechend der Inhalt des Bioreaktorbehälters eine Masse von etwa 5 g bis etwa 3000 kg aufweist. Da die Wandungen des Bioreaktorbehälters in der Regel nicht dem inneren Druck standhalten können, welcher durch die Masse des Inhalts des Bioreaktorbehälters erzeugt wird, sind Bioreaktorbehälter in der Regel in einer Bioreaktorbehälteraufnahme einer Bioreaktorvorrichtung angeordnet und darin befestigt. Dabei liegt die Wandung des Bioreaktorbehälters zumindest bereichsweise an der Wandung der Bioreaktorbehälteraufnahme der Bioreaktorvorrichtung an, so daß die Bioreaktorbehälteraufnahme der Bioreaktorvorrichtung die Wandung des Bioreaktorbehälters stützt. Die Wandungen solcher Bioreaktorbehälteraufnahmen von Bioreaktorvorrichtungen sind üblicherweise aus glattem Edelstahl ausgebildet, um eine Verunreinigung zu erschweren und eine Reinigung zu erleichtern. Ferner entspricht die Bioreaktorbehälteraufnahme der Bioreaktorvorrichtung der Bioreaktorbehälteraufnahme der Prüfvorrichtung, wobei vorzugsweise beide Bioreaktorbehälteraufnahmen identisch sind, wenn die Integritätsprüfung durchgeführt werden kann, während der Bioreaktorbehälter in der Bioreaktorvorrichtung angeordnet ist.

Wird nun nach dem Anordnen des Bioreaktorbehälters in der Bioreaktorbehälteraufnahme (der Prüfvorrichtung bzw. der Bioreaktorvorrichtung) ein Integritätstest durchgeführt, wobei das Innere des Bioreaktorbehälters mit einem Fluid beaufschlagt wird, um einen Überdruck zur Umgebung zu erzeugen, so könnte die Wandung der Bioreaktorbehälteraufnahme ein gegebenenfalls vorhandenes Leck in der Wandung des Bioreaktorbehälters abdecken bzw. abdichten.

In bzw. an der Bioreaktorbehälteraufnahme ist jedoch zumindest bereichsweise eine fluiddurchlässige und/oder eine oberflächlich strukturierte Aufnahmeschicht angeordnet. Das heißt, die Aufnahmeschicht ist zumindest bereichsweise fluiddurchlässig bzw. eine Oberfläche der Aufnahmeschicht ist strukturiert ausgebildet. Dabei ist die Aufnahmeschicht insbesondere an einer Kontaktfläche, welche ausgelegt ist, mit dem Bioreaktorbehälter zu kontaktieren, fluiddurchlässig und zwar bevorzugt in einer Richtung senkrecht zur Kontaktfläche. Bevorzugt kann die Aufnahmeschicht einen offenporigen Schaumstoff umfassen, beispielsweise aus Polyurethan. Weiter bevorzugt kann die Aufnahmeschicht aus einem im wesentlichen inkompressiblen Material bestehen, welches sich nicht durch die Aufnahme des Bioreaktorbehälters verformt, wie beispielsweise Hartschaumstoffe, Sintermaterialien, insbesondere aus Metall, poröse Keramik oder fluiddurchlässige Kunststoffe. Bevorzugt ist das Kompressionsmodul der Aufnahmeschicht größer als 10⁶ N/m² oder größer als 10⁹ N/m².

Weiter bevorzugt ist die Aufnahmeschicht einstückig ausgebildet und besteht insbesondere aus einem homogenen Material. Durch die einstückige Aufnahmeschicht kann vorteilhafterweise die Innenfläche der Bioreaktorbehälteraufnahme im wesentlichen vollständig ausgekleidet werden, wobei ein in der Bioreaktorbehälteraufnahme aufgenommener Bioreaktorbehälter die (flexible) Wandung der Bioreaktorbehälteraufnahme nicht unmittelbar kontaktiert, sondern lediglich mittelbar über die Aufnahmeschicht. Weiter bevorzugt kontaktiert die flexible Wandung des Bioreaktorbehälters neben der Aufnahmeschicht keine weiteren Teile der Bioreaktorbehälteraufnahme.

Insbesondere ist die Oberfläche der Aufnahmeschicht strukturiert, welche bei Durchführung des Verfahrens mit der Wandung des Bioreaktorbehälters kontaktiert. Bevorzugt ist die Wandung der Bioreaktorbehälteraufnahme vollständig mit der Aufnahmeschicht bedeckt. Die Aufnahmeschicht ist vorzugsweise austauschbar an bzw. in der Bioreaktorbehälteraufnahme angeordnet bzw. befestigt, so daß die Aufnahmeschicht leicht ersetzt werden kann, falls diese verschmutzt ist.

Vorteilhafterweise kann der Bioreaktorbehälter an der Wandung der Bioreaktorbehälteraufnahme mittelbar über die Aufnahmeschicht anliegen, wobei das Ergebnis des Integritätstests nicht durch das Anliegen beeinflußt wird, da das Abdichten von vorhandenen Lecks des Bioreaktorbehälters durch die Wandung der Bioreaktorbehälteraufnahme vermieden wird. Dadurch ist vorteilhafterweise eine verbesserte Integritätsprüfung möglich, durch welche Lecks in der Wandung des Bioreaktorbehälters mit größerer Sicherheit bestimmt werden können, da vermieden wird während der Integritätsprüfung ein Leck unbeabsichtigt zu verschließen.

Insbesondere kann ein Integritätstest durchgeführt werden, wenn die Bioreaktorbehälteraufnahme zu einer Bioreaktorvorrichtung gehört, mit welcher später die eigentliche Reaktion durchgeführt wird. Daher kann die Integritätsprüfung vorteilhafterweise "in situ" durchgeführt werden, vorzugsweise vor und/oder nach der durchzuführenden Reaktion ohne den Bioreaktorbehälter handhaben zu müssen, beispielsweise um den Bioreaktorbehälter in eine Integritätsprüfvorrichtung zu verbringen.

Das Diskriminieren, ob der Bioreaktorbehälter hinreichend dicht ist, kann mittels der Druckabfallmethode, durch Messung des Fluidzuflusses bei konstantem Druck oder mittels eines Testgases als Fluid erfolgen. Das Testgas ist zweckmäßigerweise ein Gas, welches nicht oder nur in Spuren in der Atmosphäre vorkommt und deshalb leicht an den Leckstellen mittels eines Gasdetektors detektieren werden kann. Der Überdruck P₁ des Fluids innerhalb des Bioreaktorbehälters gegenüber dem Atmosphärendruck beträgt zwischen etwa 20 mbar und etwa 500 mbar, bevorzugt zwischen etwa 50 mbar und etwa 300 mbar.

Die Aufnahmeschicht ist vorzugsweise porös bzw. umfaßt ein poröses Material, wobei die Porenvolumen zweckmäßigerweise untereinander derart verbunden sind, daß die Aufnahmeschicht fluiddurchlässig ist. Weiter bevorzugt kann die fluiddurchlässige Aufnahmeschicht eine richtungsabhängige bzw. anisotrope Fluiddurchlässigkeit aufweisen. Beispielsweise kann die fluidische Leitfähigkeit der Aufnahmeschicht in einer Richtung parallel zu einer Normalen der Aufnahmeschichtoberfläche größer sein als in einer Richtung senkrecht zur dieser Normalen, also parallel zur Erstreckung der Aufnahmeschichtoberfläche.

Die fluiddurchlässige Aufnahmeschicht kann metallisch ausgebildet sein. Sie kann Bestandteil der Wandung der Bioreaktorbehälteraufnahme sein.

Vorzugsweise umfaßt die fluiddurchlässige Aufnahmeschicht ein gewebtes Textil, ein nicht gewebtes Textil und/oder einen Schaumstoff. Als bevorzugte Materialien für die Aufnahmeschicht können Vliesstoffe, wie beispielsweise Spinnvliese aus Polypropylen verwendet werden. Insbesondere für die Verwendung in Reinsträumen kann die Aufnahmeschicht aus einem fusselfreien bzw. nicht staubenden Material bestehen, welches keine Partikel in die Umgebung abgibt. Ein beispielhaftes Vlies ist zum Beispiel Novatexx 2019 Viledon von der Firma Freudenberg Filtratation Technologies KG aus Polypropylen mit einem Gewicht von 17-100 g/m² und einer Luftdurchlässigkeit von 1000-5000 l/m²s bei einer Druckdifferenz von 1 bar mit einer Materialdicke von 0,25-0,75 mm. Ein weiteres beispielhaftes Material ist unter den Handelsnamen Porex® XS49020-XS49100 von der Firma Porex Technologies GmbH erhältlich. Das Material besteht aus Polypropylen und Polyethylen mit einer Materialdicke von etwa 1,5 mm bis etwa 5 mm, bevorzugt größer als etwa 3 mm. Die Größe der Poren liegt im Bereich von etwa 20 µm bis etwa 175 µm, bevorzugt kleiner als etwa 120µm. Die Luftdurchlässigkeit liegt bei etwa 150 bis etwa 300 l/cm²min bei 1,2 inch Wassersäule. Es hat sich als vorteilhaft erwiesen, für die fluiddurchlässige Außenschicht ein Polymermaterial zu verwenden, welches wärmeleitende Zusätze, wie beispielsweise Bornitrit, enthält. Dadurch läßt sich eine Temperierung des Bioreaktorbehälters vorteilhafterweise verbessern.

Die Aufnahmeschicht kann alternativ oder zusätzlich strukturiert sein. Eine strukturierte Aufnahmeschicht im Sinne der Anmeldung bedeutet, daß zumindest die Oberfläche der Aufnahmeschicht, welche bei bestimmungsgemäßem Gebrauch mit dem Bioreaktorbehälter kontaktiert, nicht glatt ist, sondern eine Struktur bzw. ein Relief aufweist. Insbesondere kann die Struktur durch Erhebungen und Vertiefungen ausgebildet werden, welche durch einen Wechsel der Materialstärke der Aufnahmeschicht erzeugt werden. Das heißt, die Aufnahmeschicht weist im Bereich von Erhebungen eine größere Materialstärke auf als im Bereich von Vertiefungen. Alternativ kann die Materialstärke der Aufnahmeschicht im wesentlichen konstant sein, wobei die Struktur in das Material geformt ist. Dabei ist insbesondere der flexibel ausgebildete Bereich der Wandung mit einer strukturierten Aufnahmeschicht versehen. Bevorzugt können aber auch starre Bereiche der Wandung einer strukturierte Aufnahmeschicht aufweisen.

Die strukturierte Aufnahmeschicht kann fluidundurchlässig sein, wodurch die Aufnahmeschicht vorteilhafterweise eine zusätzliche Abdichtfunktion erfüllen kann, um die Bioreaktorbehälteraufnahme vor Kontaminationen durch aus einem Leck auftretende Fluide zu schützen. Die strukturierte Aufnahmeschicht führt zu einem äquivalenten technischen Effekt wie eine fluiddurchlässige Aufnahmeschicht in dem Sinne, daß ein Leck in der Innenschicht nicht mittels eines Gegenstandes von außen abgedichtet werden kann. Aufgrund der Struktur der Aufnahmeschicht kann ein Gegenstand in der Regel nicht mit der Aufnahmeschicht derart fluiddicht abschließen, daß ein aus dem Bioreaktorbehälter austretendes Fluid nicht in die Umgebung gelangen würde. Insbesondere kann ein solches Abdichten nicht mittels der Bioreaktorbehälteraufnahme erfolgen. Demzufolge kann ein Integritätstest durch Beaufschlagen des Bioreaktorbehälter mit einem Überdruck erfolgen, wobei aufgrund des Lecks ein Druckabfall oder ein Fluidfluß feststellbar ist, welcher auf ein Leck schließen läßt.

Bei der Durchführung eines Integritätstest des Bioreaktorbehälters in der Bioreaktorbehälteraufnahme wird die Wandung des Bioreaktorbehälters gegen die strukturierte Aufnahmeschicht gepreßt. Die Struktur der Aufnahmeschicht führt dazu, daß fluidleitende Kanäle zwischen beiden ausgebildet werden. Dadurch kann ein durch ein Leck austretendes Fluid über die fluidleitenden Kanäle in die Umgebung geleitet werden, so daß das Leck in der Wandung des Bioreaktorbehälters nicht durch die Wandung der Bioreaktorbehälteraufnahme abdichtbar ist. Wie oben bereits beschrieben ist dadurch vorteilhafterweise eine verbesserte Integritätsprüfung möglich, durch welche Lecks in der Wandung des Bioreaktorbehälters mit größerer Sicherheit bestimmt werden können, da vermieden wird während der Integritätsprüfung ein Leck unbeabsichtigt zu verschließen, wobei weiter vorteilhafterweise die Integritätsprüfung des Bioreaktorbehälters vor und/oder nach dem Versuchsablauf bzw. dem Produktionsablauf "in situ" durchführbar ist.

Die strukturierte Aufnahmeschicht weist Vertiefungen auf, welche zumindest etwa 200 µm tief sind. Dabei definieren bzw. bilden zwei benachbarte Vertiefungen eine Erhebung. Ebenso definieren bzw. bilden zumindest zwei benachbarte Erhebungen eine Vertiefung. Weiter bevorzugt weisen die Vertiefungen gegenüber zu den benachbarten Erhebungen zumindest eine Tiefe von größer als etwa 250 µm und insbesondere von größer als etwa 500 µm auf. Dadurch wird das Ableiten des aus einem Leck austretenden Fluids in die Umgebung sichergestellt.

Die strukturierte Aufnahmeschicht weist Erhebungen auf, welche höchstens etwa 200 µm breit sind. Weiter bevorzugt weisen die Erhebungen eine Breite von weniger als etwa 150 µm, weiter bevorzugt von weniger als etwa 100 µm und insbesondere von weniger als etwa 50 µm auf. Dadurch wird verhindert, daß eine Erhebung paßgenau auf einem Leck positionierbar ist und dadurch das Leck durch eine einzige Erhebung abgedichtet wird. Der erwartete Durchmesser eines Lecks beträgt etwa 5 µm bis etwa 1000 µm.

Vorzugsweise sind die Erhebungen und/oder Vertiefungen der strukturierten Aufnahmeschicht entlang einer Vorzugsrichtung V orientiert. Insbesondere erstrecken sich die Erhebungen und/oder Vertiefungen im wesentlichen entlang einer Längsrichtung, wobei benachbarte Erhebungen und/oder Vertiefungen parallel zueinander orientiert sind. Die Vorzugsrichtung V entspricht dabei der Längsrichtung entlang welcher sich die Erhebungen bzw. Vertiefungen erstrecken. Mit anderen Worten können die Erhebungen und Vertiefungen insbesondere eine Rillenstruktur oder eine Rautenstruktur auf der Aufnahmeschicht ausbilden.

Vorzugsweise umfaßt das Bereitstellen der Bioreaktorbehälteraufnahme und der fluiddurchlässigen oder strukturierten Aufnahmeschicht, daß die Aufnahmeschicht an bzw. in der Bioreaktorbehälteraufnahme angeordnet wird. Beispielsweise kann die Bioreaktorbehälteraufnahme mit der Aufnahmeschicht ausgekleidet werden. Insbesondere kann die Aufnahmeschicht bereits in einer zu der Bioreaktorbehälteraufnahme formkongruenten Gestalt bereitgestellt werden, so daß die Aufnahmeschicht lediglich in die Bioreaktorbehälteraufnahme eingelegt werden muß, wobei insbesondere die Bioreaktorbehälteraufnahme im wesentlichen nicht von der Aufnahmeschicht beabstandet ist. Um die Formkongruenz der Aufnahmeschicht zu erreichen kann die Aufnahmeschicht aus mehreren Teilen zusammengefügt sein, beispielsweise durch Kleben, Verschweißen oder Nähen.

Vorzugsweise umfaßt das Verfahren die anschließenden Schritte:
- Füllen des Bioreaktorbehälters mit Edukten;
- Durchführen einer chemischen bzw. biochemischen Reaktion in dem Bioreaktorbehälter;
- Ablassen des Inhalts aus dem Bioreaktorbehälter.

Vorteilhafterweise kann der Integritätstest in das Produktionsverfahren integriert werden, da direkt im Anschluß an den Integritätstest der Bioreaktorbehälter mit den Edukten bzw. Ausgangsstoffen der Reaktion befüllt werden kann. Um das restliche für den Integritätstest verwendete Fluid aus dem Bioreaktorbehälter zu entfernen, können die Ausgangsstoffe zweckmäßigerweise über eine am Bioreaktorbehälter unten gelegene Behälteröffnung zugeführt werden, während das Fluid über eine oben gelegene Behälteröffnung entlüftet wird, insbesondere über einen Sterilfilter. Insbesondere bei proteinhaltigen Ausgangsstoffen wird ein Aufschäumen vorteilhafterweise vermieden.

Vorzugsweise erfolgt das Diskriminieren, ob der Bioreaktorbehälter hinreichend dicht ist, vor und/oder nach dem Durchführen der biochemischen Reaktion in dem Bioreaktorbehälter. Mit anderen Worten umfaßt das Verfahren bevorzugt die an das Ablassen anschließenden Schritte:
- Verbinden der zumindest einen Behälteröffnung des Bioreaktorbehälters mit einer Fluidquelle;
- Füllen des Bioreaktorbehälters mit einem Fluid aus der Fluidquelle, um zu einem ersten Zeitpunkt T₁ einen vorbestimmten Überdruck P₁ in dem Bioreaktorbehälter zu erzeugen, wobei die Wandung des Bioreaktorbehälters zumindest bereichsweise die Aufnahmeschicht der Bioreaktorbehälteraufnahme kontaktiert;
- Diskriminieren, ob der Bioreaktorbehälter hinreichend dicht ist.

Das Verbinden der zumindest einen Behälteröffnung des Bioreaktorbehälters erfolgt mit der Fluidquelle erfolgt bevorzugt über einen Sterilfilter.

Das Diskriminieren kann wie oben bereits beschrieben anhand einer Druckdifferenz (P₂-P₁) zwischen einem Überdruck P₂ zu einem späteren Zeitpunkt T₂ und dem bestimmten Überdruck zu Zeitpunkt T₁, oder anhand einer Fluidmenge M, welche nach dem Zeitpunkt T₁ dem Bioreaktorbehälter zugeführt wurde, um den Überdruck P₁ konstant zu halten, oder anhand einer Detektion von Fluidstoffen bzw. Fluidpartikeln außerhalb oder innerhalb des Bioreaktorbehälters, welche dem Bioreaktorbehälter zugeführt wurden, erfolgen.

Mit anderen Worten kann vorteilhafterweise nach Beendigung der Reaktion ein abschließender Integritätstest durchgeführt werden, um zu überprüfen, ob der Bioreaktorbehälter über den gesamten Zeitraum der Reaktion seine Integrität behalten hat.

### Vorrichtung zum Prüfen der Integrität eines Bioreaktorbehälters

Ein Aspekt betrifft eine Vorrichtung zum Prüfen der Integrität eines Bioreaktorbehälters umfassend:
- eine Bioreaktorbehälteraufnahme zur Aufnahme eines Bioreaktorbehälters;
- zumindest eine fluiddurchlässige und/oder strukturierte Aufnahmeschicht, welche austauschbar zumindest teilweise an der Bioreaktorbehälteraufnahme angeordnet ist, wobei die Aufnahmeschicht ausgelegt ist, zumindest bereichsweise zwischen einer Wandung des Bioreaktorbehälters und der Bioreaktorbehälteraufnahme angeordnet zu sein.

Die Bioreaktorbehälteraufnahme ist im wesentlichen starr. Ein Bioreaktorbehälter kann entlang einer Einführrichtung E in die Bioreaktorbehälteraufnahme eingeführt werden und ist durch die Bioreaktorbehälteraufnahme derart aufgenommen, daß eine weitere Verlagerung des Bioreaktorbehälters entlang der Einführrichtung E und bevorzugt auch senkrecht zur Einführrichtung E gehemmt ist. Weiter bevorzugt begrenzt die Bioreaktorbehälteraufnahme die volumetrische Ausdehnung des Bioreaktorbehälters bei bzw. nach dessen Befüllung. Bevorzugt weist die Bioreaktorbehälteraufnahme zumindest eine Sichtöffnung auf, durch welche der Bioreaktorbehälter in der Bioreaktorbehälteraufnahme sichtbar ist. Die Sichtöffnung kann als durchgehende Öffnung in der Wandung der Bioreaktorbehälteraufnahme ausgebildet sein. Alternativ kann die Sichtöffnung durch ein durchsichtiges Material verschlossen sein. Vorteilhafterweise kann das Bioreaktorbehälter und insbesondere das Innere des Bioreaktorbehälters in geschlossenem Zustand während des betriebsgemäßen Gebrauchs kontinuierlich visuell inspiziert werden.

Bevorzugt ist der Bereich der Wandung der Bioreaktorbehälteraufnahme, welcher bei betriebsgemäßem Gebrauch mit dem Bioreaktorbehälter kontaktiert bzw. welche den Wandungsdruck des Bioreaktorbehälters aufnimmt, die fluiddurchlässige und/oder strukturierte Aufnahmeschicht angeordnet. Insbesondere ist die Wandung der Bioreaktorbehälteraufnahme vollständig mit der fluiddurchlässigen und/oder strukturierten Aufnahmeschicht bedeckt. Mit anderen Worten wird der Bioreaktorbehälter mittelbar über die Aufnahmeschicht in der Bioreaktorbehälteraufnahme aufgenommen.

Die Aufnahmeschicht ist austauschbar an der Bioreaktorbehälteraufnahme angeordnet bzw. befestigt. Vorteilhafterweise kann die Aufnahmeschicht im Falle einer Kontamination in einfacher Weise ausgetauscht werden. Das Befestigen der Aufnahmeschicht kann durch Befestigungsmittel erfolgen, welche an der Aufnahmeschicht befestigt sind, und in Kräftschluß bzw. Eingriff mit komplementären Befestigungsmitteln der Bioreaktorbehälteraufnahme stehen. Beispielsweise können die Aufnahmeschicht und die Bioreaktorbehälteraufnahme komplementäre Teile eines Klettverschlusses aufweisen. Alternativ oder zusätzlich kann die Bioreaktorbehälteraufnahme Vorsprünge aufweisen, welche in Öffnungen der Aufnahmeschicht eingreifen. Alternativ oder zusätzlich kann die Aufnahmeschicht einen Kragen aufweisen, welcher über einen Randbereich der Bioreaktorbehälteraufnahme stülpbar ist. Zu bevorzugten Materialien der Aufnahmeschicht wird auf die Beschreibung des Verfahrens verwiesen.

Vorzugsweise umfaßt die Vorrichtung eine Fluidquelle, welche mit einer Behälteröffnung des Bioreaktorbehälters fluidisch verbindbar ist. Nach dem Verbinden der zumindest einen Behälteröffnung des Bioreaktorbehälters mit der Fluidquelle ist der Bioreaktorbehälters mit einem Fluid aus der Fluidquelle füllbar, um einen vorbestimmten Überdruck P₁ in dem Bioreaktorbehälter zu erzeugen. Durch den Überdruck P₁ wird die Wandung des Bioreaktorbehälters gegen die Wandung der Bioreaktorbehälteraufnahme gepreßt, so daß die Aufnahmeschicht zumindest bereichsweise zwischen dem Bioreaktorbehälter und der Bioreaktorbehälteraufnahme festgeklemmt bzw. festgepreßt ist.

Vorzugsweise umfaßt die Vorrichtung einen Drucksensor, welche den Fluiddruck innerhalb des Bioreaktorbehälters erfassen kann. Bevorzugt können die Fluidquelle und der Drucksensor gemeinsam in einer Prüfeinrichtung angeordnet sein. Die Prüfeinrichtung kann insbesondere eine automatische Prüfeinrichtung sein, welche die Regelung der Fluidquelle und die Erfassung des Druckes mittels der Drucksensors automatisch bzw. computergestützt durchführt.

Die Prüfeinrichtung kann diskriminieren, ob der Bioreaktorbehälter (1) hinreichend dicht ist. Dabei kann das Diskriminieren insbesondere anhand einer Druckdifferenz (P₂-P₁) zwischen einem Überdruck P₂ zu einem späteren Zeitpunkt T₂ und dem bestimmten Überdruck P₁ zu Zeitpunkt T₁, erfolgen, wobei die Drücke durch den Drucksensor erfaßt werden. Alternativ oder zusätzlich kann das Diskriminieren anhand einer Fluidmenge M erfolgen, welche nach dem Zeitpunkt T₁ dem Bioreaktorbehälter (1) zugeführt werden mußte, um den Überdruck P₁ konstant zu halten. Zweckmäßigerweise umfaßt die Prüfvorrichtung dann auch eine Fluidmengenerfassungseinrichtung, welche die Menge von Fluid erfaßt, welche dem Bioreaktorbehälter zugeführt wird. Zusätzlich können Fluidstoffe bzw. Fluidpartikeln außerhalb oder innerhalb des Bioreaktorbehälters detektiert werden, welche dem Bioreaktorbehälter zugeführt wurden. Zweckmäßigerweise wird der Bioreaktorbehälter dann mit einem Testgas gefüllt, wobei die Prüfvorrichtung dann auch bevorzugt einen Gasdetektor umfaßt, welcher bezüglich Spuren dieses Testgases sensitiv ist. Es versteht sich, daß auch ein externer Gasdetektor bereitgestellt sein kann.

### Verwendung einer fluiddurchlässigen und/oder strukturierten Aufnahmeschicht

Ein Aspekt betrifft eine Verwendung einer fluiddurchlässigen und/oder strukturierten Aufnahmeschicht zum austauschbaren Anordnen an einer Bioreaktorbehälteraufnahme, wobei die Aufnahmeschicht nach dem Anordnen eines Bioreaktorbehälters in der Bioreaktorbehälteraufnahme zumindest bereichsweise durch den Bioreaktorbehälter und der Bioreaktorbehälteraufnahme zwischen diesen festgeklemmt ist.

In weiteren bevorzugten Ausführungsformen der Erfindung umfaßt die fluiddurchlässige Aufnahmeschicht ein gewebtes Textil, ein nicht gewebtes Textil und/oder einen Schaumstoff. Die strukturierte Aufnahmeschicht weist Vertiefungen auf, welche zumindest etwa 200 µm tief sind und/oder weist die strukturierte Aufnahmeschicht Erhebungen auf, welche höchstens etwa 200 µm breit sind. Die bevorzugten Ausführungsformen der Erfindung betreffend dabei insbesondere Ausführungsformen des Verfahrens, der Vorrichtung und der Verwendung.

### Figurenbeschreibung

Nachfolgend werden bevorzugte Ausführungsformen der vorliegenden Erfindung anhand der beigefügten Zeichnungen beispielhaft erläutert. Einzelne Merkmale der gezeigten bevorzugten Ausführungsformen können zu weiteren bevorzugten Ausführungsformen kombiniert werden. Es zeigen:
- Figur 1: eine perspektivische Ansicht eines Bioreaktorbehälters und einer Bioreaktorbehälteraufnahme;
- Figur 2a: einen Schnitt durch eine Ausführungsform einer strukturierten Aufnahmeschicht;
- Figur 2b: eine Draufsicht auf die strukturierte Aufnahmeschicht;
- Figur 3a: einen Schnitt durch eine weitere Ausführungsform einer strukturierten Aufnahmeschicht;
- Figur 3b: eine Draufsicht auf die strukturierte Aufnahmeschicht;
- Figur 4a: einen Schnitt durch eine weitere Ausführungsform einer strukturierten Aufnahmeschicht;
- Figur 4b: eine Draufsicht auf die strukturierte Aufnahmeschicht;
- Figur 5: eine schematische Ansicht einer Vorrichtung zum Prüfen der Integrität des Bioreaktorbehälters.

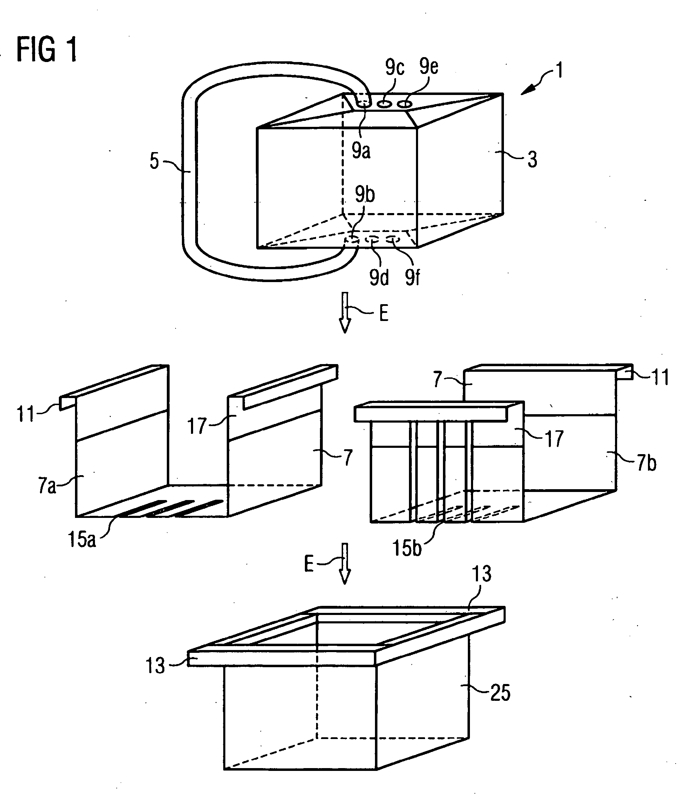

Die **Figur 1** zeigt eine bevorzugte Ausführungsform eines Bioreaktorbehälters 1 einer Bioreaktorbehälteraufnahme 25 in einer perspektivischen Ansicht. Der Bioreaktorbehälter 1 ist insbesondere ein flexibler Behälter, wie zum Beispiel ein Beutel, der für die Prozeßführung bzw. Lagerung von biotechnologischen Produkten verwendet werden kann. Der Bioreaktorbehälter 1 kann dabei bereichsweise flexible Wände aufweisen. Beispielsweise kann ein Bioreaktorbehälter 1 ein Fermenter sein. Der Bioreaktorbehälter 1 dient dabei bevorzugt zur Aufnahme, zum Mischen, zur Speicherung und zum Spenden von sterilen Medien. Insbesondere kann der Bioreaktorbehälter 1 ausgelegt sein, Fluide unter sterilen Bedingung aufzunehmen und abzugeben, beispielsweise mittels geeigneter Sterilfilter.

Der gezeigte Bioreaktorbehälter 1 weist eine quaderförmige bzw. würfelförmige Gestalt auf. Es versteht sich, daß der Bioreaktorbehälter 1 auch eine tetraederförmige, eine zylindrische, eine kugelförmige, eine prismenförmige oder eine sonstige beliebige Gestalt aufweisen kann. Diesbezüglich sein angemerkt, daß der Begriff "Bioreaktorbehälter" im Sinne der Erfindung gleichermaßen Bioreaktoren sowie Behälter mit bereichsweise flexiblen Wänden umfaßt, die beispielsweise zur Aufnahme, zum Mischen, zur Speicherung und zum Spenden von sterilen Medien dienen. Der Bioreaktorbehälter 1 umfaßt eine fluidundurchlässige, flexible Wandung 3, die vorzugsweise aus einer Folie bzw. aus einem Laminat mehrerer Folien besteht. Mit anderen Worten kann die Wandung 3 im wesentlichen als flexibler Beutel ausgebildet sein, welcher in der Gestalt variieren kann.

Das Innenvolumen des Bioreaktorbehälters 1 ist über die Behälteröffnungen 9a, 9b, 9c, 9d, 9e, 9f mit der Umgebung oder mit weiteren Elementen, wie zum Beispiel Fluidleitungen, fluidisch verbindbar bzw. verbunden. Es versteht sich, daß die fluidundurchlässige, flexible Wandung 3 im Bereich der Behälteröffnungen 9a, 9b, 9c, 9d, 9e, 9f versteift bzw. starr ausgebildet sein kann, damit die 9a, 9b, 9c, 9d, 9e, 9f formstabil sind und gegebenenfalls daran anschließende Anschlüsse und Verbinder dicht bleiben. Zwei der Behälteröffnungen 9a, 9b können miteinander durch eine Fluidleitung 5 fluidisch verbunden sein, so daß ein Umwälzen des Inhalts des Bioreaktorbehälters 1 durch Transportieren von Fluid von einer der Behälteröffnungen 9b zu der anderen einer der Behälteröffnungen 9a über die Fluidleitung 5 möglich ist. Das Umwälzen erfolgt bevorzugt mittels einer Umwälzpumpe (nicht dargestellt). Über die verbleibenden Behälteröffnungen 9c, 9d, 9e, 9f kann der Bioreaktorbehälter 1 beispielsweise befüllt und entleert werden.

Die in Figur 1 gezeigte Bioreaktorbehälteraufnahme 25 ist im wesentlichen starr und bevorzugt aus einem korrosionsbeständigen Material hergestellt, wie beispielsweise Edelstahl. In die Bioreaktorbehälteraufnahme 25 kann entlang einer Einführrichtung E eine fluiddurchlässige und/oder strukturierte Aufnahmeschicht 7 eingeführt werden, wobei die Aufnahmeschicht 7 in der gezeigten Ausführungsform zwei Lagen 7a, 7b umfaßt, welche übereinander angeordnet werden können. Der Bioreaktorbehälter 1 kann ebenfalls entlang der Einführrichtung E in die Bioreaktorbehälteraufnahme 25 eingeführt werden. Nach dem Einführen ist der Bioreaktorbehälter 1 durch die Bioreaktorbehälteraufnahme 25 derart aufgenommen, daß eine weiter Verlagerung des Bioreaktorbehälters entlang der Einführrichtung E und auch senkrecht zu Einführrichtung E gehemmt ist. Die starre Bioreaktorbehälteraufnahme 25 begrenzt weiter die volumetrische Ausdehnung des Bioreaktorbehälters 1 bei bzw. nach dessen Befüllung.

Nach dem Anordnen der Aufnahmeschicht 7 und des Bioreaktorbehälters 1 in der Bioreaktorbehälteraufnahme 25 ist die Aufnahmeschicht 7 zumindest bereichsweise zwischen der Bioreaktorbehälteraufnahme 25 und der Wandung 3 des Bioreaktorbehälters 1 festgeklemmt, insbesondere aufgrund des Druckes, welcher der Bioreaktorbehälters 1 auf die Bioreaktorbehälteraufnahme 25 ausübt. Bevorzugt ist die Aufnahmeschicht 7 derart in der Bioreaktorbehälteraufnahme 25 angeordnet, daß die der Wandung 3 des Bioreaktorbehälters 1 die Bioreaktorbehälteraufnahme 25 bei betriebsgemäßem Gebrauch, also insbesondere nach dem Befüllen des Bioreaktorbehälters 1 mit einem Fluid, nicht unmittelbar kontaktiert. Dahingegen kontaktiert der Bioreaktorbehälter 1 die Aufnahmeschicht 7. Insbesondere sind die Wandungen der Bioreaktorbehälteraufnahme 25, welche dem Bioreaktorbehälter 1 zugewandt sind, vollständig mit der fluiddurchlässigen und/oder strukturierten Aufnahmeschicht 7 bedeckt.

Die Aufnahmeschicht 7 ist austauschbar an der Bioreaktorbehälteraufnahme 25 angeordnet bzw. befestigt, so daß Aufnahmeschicht 7 in einfacher Weise ausgetauscht werden kann. Das Befestigen der Aufnahmeschicht 7 kann durch Befestigungsmittel erfolgen, welche an der Aufnahmeschicht 7 befestigt sind, und in Kraftschluß bzw. Eingriff mit komplementären Befestigungsmitteln der Bioreaktorbehälteraufnahme 25 stehen. Die in Figur 1 gezeigte Ausführungsform der Aufnahmeschicht 7 weist einen Kragen 11 auf, welcher über einen Randbereich 13 der Bioreaktorbehälteraufnahme 25 stülpbar ist. Der Kragen 11 kann beispielsweise durch eine Materialspannung in der Aufnahmeschicht 7 in seiner Position an dem Randbereich 13 der Bioreaktorbehälteraufnahme 25 gehalten sein, beispielsweise durch einen Bereich der Aufnahmeschicht 7, welcher elastisch dehnbar ausgeführt ist, oder durch einen Gummizug. Alternativ oder zusätzlich kann die Aufnahmeschicht 7 durch weitere Befestigungsmittel mit der Bioreaktorbehälteraufnahme 25 lösbar verbunden sein. Bewährt haben sich Klettverschlüsse als bevorzugtes Befestigungsmittel.

Die Aufnahmeschicht 7 kann einteilig ausgebildet sein oder wie in der Figur 1 gezeigt aus mehreren Teilen bzw. Lagen 7a, 7b zusammengesetzt sein. Je nach der Form der Bioreaktorbehälteraufnahme 25 kann eine einteilige oder mehrteilige Aufnahmeschicht 7 eine einfachere Handhabung aufweisen. In der in Figur 1 gezeigten Ausführungsform der Aufnahmeschicht 7 werden zwei rechteckige Lagen 7a, 7b kreuzweise übereinander entlang der Einführrichtung E in die Bioreaktorbehälteraufnahme 25 eingeführt, so daß in einfacher Weise der Kragen 11 über den Randbereich 13 stülpbar ist. Die Aufnahmeschicht 7 kann insbesondere Durchtritte 15a, 15b aufweisen. Die Durchtritte 15a, 15b können runde Öffnungen oder Schlitze sein, damit beispielsweise ein Fluidleitung 5 durch die Aufnahmeschicht 7 zu einer der Behälteröffnungen 9a bis 9f geführt werden kann.

Die Aufnahmeschicht 7 kann unabhängig von der äußeren Gestalt starr oder flexibel ausgebildet sein. Vorteilhafterweise ermöglicht eine flexible Aufnahmeschicht 7, daß die Aufnahmeschicht 7 in einfacher Weise zusammenlegbar und platzsparend lagerbar ist. Die Aufnahmeschicht 7 kann auch nur bereichsweise starr ausgebildet sein bzw. steifer als in anderen Bereichen ausgebildet sein. Beispielsweise kann ein Verstärkungsbereich 17 in Bereich des Kragen 11 und/oder im Bereich der Durchtritte 15a, 15b vorgesehen sein.

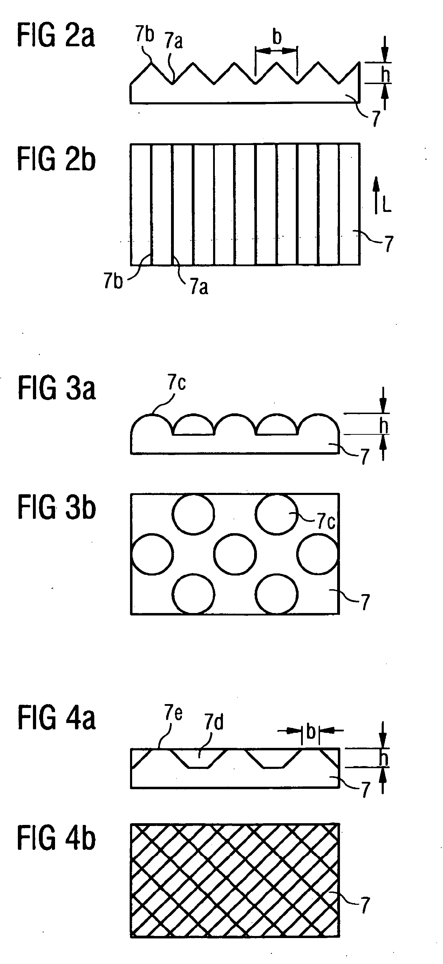

Die **Figur 2a** zeigt einen Schnitt durch und die **Figur 2** **b** eine Draufsicht auf eine Ausführungsform einer strukturierten Aufnahmeschicht 7. Die Aufnahmeschicht 7 weist in dieser Ausführungsform Vertiefungen 7a und Erhebungen 7b auf, welche sich parallel zueinander entlang einer Längsrichtung L erstrecken. Mit anderen Worten bilden die Erhebungen 7b und Vertiefungen 7a eine Rillenstruktur auf der Aufnahmeschicht 7 bzw. der Außenoberfläche aus. Die Erhebungen 7b und Vertiefungen 7a werden durch einen Wechsel der Materialstärke der Aufnahmeschicht 7 ausgebildet. Vorzugsweise umfaßt die strukturierte Außenseite Erhebungen, welche eine Breite b von höchstens etwa 200 µm, bevorzugt etwa 50 µm und eine Höhe h von höchstens etwa 200 µm, bevorzugt etwa 50 µm, aufweisen.

Die **Figur 3a** zeigt einen Schnitt durch und die **Figur 3** **b** eine Draufsicht auf eine Ausführungsform einer strukturierten Aufnahmeschicht 7. Die Aufnahmeschicht 7 weist in dieser Ausführungsform halbkugelförmige Erhebungen 7c bzw. Noppen 7c auf. Die Noppen 7c sind vorzugsweise regelmäßig auf der Aufnahmeschicht 7 angeordnet. Die Erhebungen bzw. Noppen 7c weisen bevorzugt eine Höhe h von höchstens etwa 200 µm auf. Die Punktauflage sollte kleiner als 5 µm, bevorzugt kleiner als 2,5 µm sein, zumindest aber höchstens den halben Durchmesser der zu detektierenden Lochgröße sein.

Die **Figur 4a** zeigt einen Schnitt durch und die **Figur 4** **b** eine Draufsicht auf eine Ausführungsform einer strukturierten Aufnahmeschicht 7. Die Aufnahmeschicht 7 weist in dieser Ausführungsform Vertiefungen 7d und Erhebungen 7e auf, welche eine Rautenstruktur auf der Aufnahmeschicht 7 bzw. deren Oberfläche ausbilden. Die Erhebungen 7e und Vertiefungen 7d werden durch einen Wechsel der Materialstärke der Aufnahmeschicht 7 ausgebildet, wobei die Erhebungen eine Breite b von höchstens etwa 5 µm, bevorzugt etwa 2,5 µm und eine Höhe h von zumindest etwa 100 µm aufweisen.

Es versteht sich, das die Erhebungen und Vertiefungen der Aufnahmeschicht 7 kein regelmäßiges bzw. periodisches Muster ausbilden müssen, sondern eine ungeordnete, unregelmäßige Struktur ausbilden können.

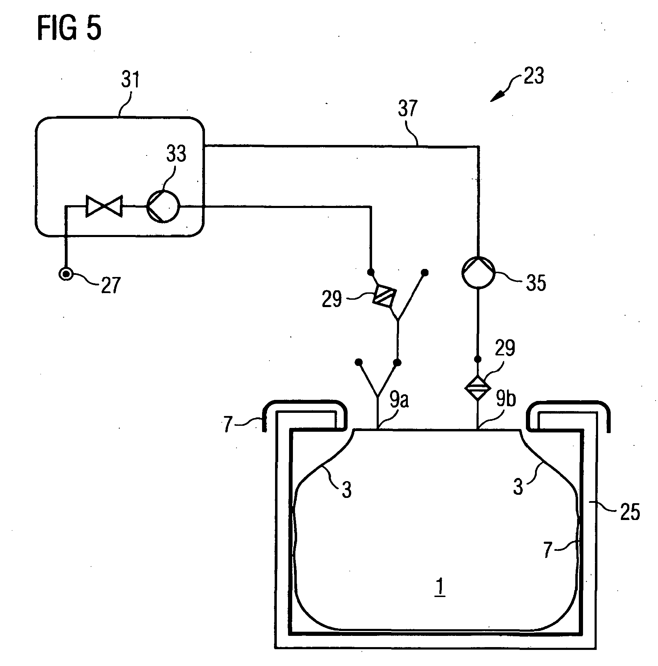

Die **Figur 5** zeigt eine schematische Ansicht einer Anordnung 23 zum Prüfen der Integrität des Bioreaktorbehälters 1. Zum Prüfen wird der Bioreaktorbehälter 1 in einer Bioreaktorbehälteraufnahme 25 einer Bioreaktorvorrichtung angeordnet. Eine Aufnahmeschicht 7 befindet sich nach dem Anordnen zwischen der Bioreaktorbehälteraufnahme 25 und einer Wandung 3 des Bioreaktorbehälters 1. Zumindest eine Behälteröffnung 9a des Bioreaktorbehälters 1 wird mit einer Fluidquelle 27 fluidisch verbunden, beispielsweise über einen Sterilfilter 29, um das Innere des Bioreaktorbehälters 1 steril zu halten. Die Fluidquelle kann Teil einer Prüfvorrichtung 31, wie z.B. Sartocheck® 4plus der Sartorius Stedium Biotech GmbH, sein oder eine externe Fluidquelle 27 sein.

Der Bioreaktorbehälter 1 wird mit einem Fluid aus der Fluidquelle 27 zu einem Zeitpunkt T₁ mit einem vorbestimmten Überdruck P₁ von etwa 50 mbar bis etwa 100 mbar gefüllt, wobei die Wandung 3 des Bioreaktorbehälters 1 zumindest bereichsweise an die Aufnahmeschicht 7 und diese dann gegen die Bioreaktorbehälteraufnahme 25 gepreßt wird. Dadurch wird die Aufnahmeschicht 7 zwischen dem Bioreaktorbehälter 1 und der Bioreaktorbehälteraufnahme 25 festgeklemmt, wobei die Aufnahmeschicht 7 ein unmittelbares Kontaktieren des Bioreaktorbehälters 1 mit der Bioreaktorbehälteraufnahme 25 verhindert.

Die Druckdifferenz P₂-P₁ zwischen einem Überdruck P₂- zu einem späteren Zeitpunkt T₂ und dem bestimmten Überdruck zu Zeitpunkt T₁ kann mittels eines internen Drucksensors 33 der Prüfvorrichtung 31 erfaßt werden oder mittels eines externen Drucksensors 35, welcher mit einer Behälteröffnung 9b fluidisch verbunden ist. Der externe Drucksensor 35 ist bevorzugt mit der Prüfvorrichtung 31 über eine Signalleitung 37 verbunden. Anhand der Druckdifferenz P₂-P₁ kann bestimmt werden, ob der Bioreaktorbehälter 1 dicht bzw. integer ist. Dazu muß die Druckdifferenz kleiner als ein vorbestimmter Betrag sein, im Idealfall gleich null. Vorteilhafterweise kann der Bioreaktorbehälter 1 während der Prüfung an der Aufnahmeschicht 7 in der Bioreaktorbehälteraufnahme 25 anliegen, wobei das Ergebnis des Integritätstests nicht beeinflußt wird, da das Abdichten von vorhandenen Lecks durch die Aufnahmeschicht 7 vermieden wird.

### Bezugszeichenliste

- 1: Bioreaktorbehälter
- 3: Wandung
- 5: Fluidleitung
- 7: Aufnahmeschicht
- 9a-f: Behälteröffnung
- 11: Kragen der Aufnahmeschicht
- 13: Randbereich der Bioreaktorbehälteraufnahme
- 15a, b: Durchtrittsöffnung
- 17: Verstärkungsbereich
- 23: Anordnung zum Prüfen
- 25: Bioreaktorbehälteraufnahme
- 27: Fluidquelle
- 29: Sterilfilter
- 31: Prüfvorrichtung
- 33: interner Drucksensor
- 35: externer Drucksensor
- 37: Signalleitung
- E: Einführrichtung
- L: Längsrichtung

## Patentansprüche

1. Verfahren zum Prüfen der Integrität eines Bioreaktorbehälters (1) mit den Schritten:
- Bereitstellen eines Bioreaktorbehälters (1) mit einer fluiddichten, zumindest bereichsweise flexiblen Wandung (3) und mit zumindest einer Behälteröffnung (9a, 9b, 9c, 9d);
- Bereitstellen einer Prüfvorrichtung mit einer Bioreaktorbehälteraufnahme (25) und einer fluiddurchlässigen und/oder strukturierten Aufnahmeschicht (7), welche austauschbar an der Bioreaktorbehälteraufnahme (25) angeordnet ist, wobei die strukturierte Aufnahmeschicht Vertiefungen aufweist, welche zumindest etwa 200 µm tief sind und/oder wobei die strukturierte Aufnahmeschicht Erhebungen aufweist, welche höchstens etwa 200 µm breit sind;
- Anordnen des Bioreaktorbehälters (1) zumindest teilweise in der Bioreaktorbehälteraufnahme (25), wobei der Bioreaktorbehälter (1) mit seiner Wandung (3) zumindest bereichsweise die Aufnahmeschicht (7) in der Bioreaktorbehälteraufnahme (25) kontaktiert;
- Verbinden der zumindest einen Behälteröffnung (9a, 9b, 9c, 9d) des Bioreaktorbehälters mit einer Fluidquelle (27);
- Füllen des Bioreaktorbehälters (1) mit einem Fluid aus der Fluidquelle (27), um zu einem ersten Zeitpunkt T₁ einen vorbestimmten Überdruck P₁ in dem Bioreaktorbehälter (1) zu erzeugen;
- Diskriminieren, ob der Bioreaktorbehälter (1) hinreichend dicht ist.

2. Verfahren gemäß Anspruch 1, wobei das Diskriminieren, ob der Bioreaktorbehälter (1) hinreichend dicht ist, anhand einer Druckdifferenz (P₂-P₁) zwischen einem Überdruck P₂ zu einem späteren Zeitpunkt T₂ und dem bestimmten Überdruck zu Zeitpunkt T₁ erfolgt.

3. Verfahren gemäß Anspruch 1 oder 2, wobei das Diskriminieren, ob der Bioreaktorbehälter (1) hinreichend dicht ist, anhand einer Fluidmenge M, welche nach dem Zeitpunkt T₁ dem Bioreaktorbehälter (1) zugeführt wurde, um den Überdruck P₁ konstant zu halten erfolgt.

4. Verfahren gemäß einem der vorigen Ansprüche, wobei das Diskriminieren, ob der Bioreaktorbehälter (1) hinreichend dicht ist, anhand einer Detektion von Fluidstoffen, welche dem Bioreaktorbehälter (1) zugeführt wurden, außerhalb oder innerhalb des Bioreaktorbehälters (1) erfolgt.

5. Verfahren gemäß einem der vorigen Ansprüche, weiter umfassend die Schritte:
- Füllen des Bioreaktorbehälters (1) mit Edukten;
- Durchführen einer chemischen bzw. biochemischen Reaktion in dem Bioreäktorbehälter (1);
- Ablassen des Inhalts aus dem Bioreaktorbehälter (1).

6. Verfahren gemäß einem der vorigen Ansprüche, wobei das Diskriminieren, ob der Bioreaktorbehälter (1) hinreichend dicht ist, vor und nach dem Durchführen der biochemischen Reaktion in dem Bioreaktorbehälter (1) erfolgt.

7. Verfahren gemäß einem der vorigen Ansprüche, wobei die fluiddurchlässige Aufnahmeschicht ein gewebtes Textil, ein nicht gewebtes Textil und/oder einen Schaumstoff umfaßt.

8. Vorrichtung zum Prüfen der Integrität eines Bioreaktorbehälters umfassend:
- eine Bioreaktorbehälteraufnahme (25) zur Aufnahme eines Bioreaktorbehälters (1);
- zumindest eine fluiddurchlässige und/oder strukturierte Aufnahmeschicht (7), welche austauschbar zumindest teilweise an der Bioreaktorbehälteraufnahme (25) angeordnet ist, wobei die Aufnahmeschicht (7) ausgelegt ist, zumindest bereichsweise zwischen einer Wandung (3) des Bioreaktorbehälters (1) und der Bioreaktorbehälteraufnahme (25) angeordnet zu sein, und wobei die strukturierte Aufnahmeschicht Vertiefungen aufweist, welche zumindest etwa 200 µm tief sind und/oder wobei die strukturierte Aufnahmeschicht Erhebungen aufweist, welche höchstens etwa 200 µm breit sind; und
- eine Prüfeinrichtung (31), durch welche diskriminierbar ist, ob der Bioreaktorbehälter (1) hinreichend dicht ist,
wobei das Diskriminieren anhand einer Druckdifferenz (P₂-P₁) zwischen einem Überdruck P₂ zu einem späteren Zeitpunkt T₂ und dem bestimmten Überdruck zu Zeitpunkt T₁,
und/oder
anhand einer Fluidmenge M, welche nach dem Zeitpunkt T₁ dem Bioreaktorbehälter (1) zugeführt wurde, um den Überdruck P₁ konstant zu halten, erfolgt.

9. Vorrichtung gemäß Anspruch 8, weiter umfassend:
- eine Fluidquelle (27), welche mit einer Behälteröffnung (9a, 9b, 9c, 9d) des Bioreaktorbehälters (1) fluidisch verbindbar ist.

10. Vorrichtung gemäß Anspruch 8 oder 9,
wobei das Diskriminieren ferner anhand einer Detektion von Fluidstoffen, welche dem Bioreaktorbehälter (1) zugeführt wurden, außerhalb des Bioreaktorbehälters (1), erfolgt.

11. Vorrichtung gemäß einem der Ansprüche 8 bis 10, wobei die fluiddurchlässige Aufnahmeschicht ein gewebtes Textil, ein nicht gewebtes Textil und/oder einen Schaumstoff umfaßt.

12. Verwendung einer fluiddurchlässigen und/oder strukturierten Aufnahmeschicht (7) zum austauschbaren Anordnen an einer Bioreaktorbehälteraufnahme (25) einer Vorrichtung zum Prüfen der Integrität eines Bioreaktorbehälters gemäß einem der Ansprüche 8 bis 11, wobei die Aufnahmeschicht (7) nach dem Anordnen eines Bioreaktorbehälters (1) in der Bioreaktorbehälteraufnahme (25) zumindest bereichsweise durch den Bioreaktorbehälter und der Bioreaktorbehälteraufnahme (25) zwischen diesen festgeklemmt ist.

## Claims

1. Method for testing the integrity of a bioreactor container (1) including the steps:
- providing a bioreactor container (1) having a fluid-tight, at least in some areas flexible wall (3) and having at least one container opening (9a, 9b, 9c, 9d);
- providing a testing apparatus having a bioreactor container reception (25) and a fluid-permeable and/or structured reception layer (7), which is replaceably arranged at the bioreactor container reception (25), wherein the structured reception layer has recesses having a depth of at least approximately 200 µm and/or wherein the structured reception layer has elevations having a width of at most approximately 200 µm;
- arranging the bioreactor container (1) at least partially in the bioreactor container reception (25), wherein the bioreactor container (1) at least in some areas contacts the reception layer (7) in the bioreactor container reception (25) with its wall (3);
- connecting the at least one container opening (9a, 9b, 9c, 9d) of the bioreactor container to a fluid source (27);
- filling the bioreactor container (1) with a fluid from the fluid source (27) in order to generate a predetermined overpressure P₁ in the bioreactor container (1) at a first point in time T₁;
- discriminating whether the bioreactor container (1) is sufficiently tight.

2. Method according to claim 1, wherein discriminating whether the bioreactor container (1) is sufficiently tight is effected by means of a pressure difference (P₂-P₁) between an overpressure P₂ at a later point in time T₂ and the predetermined overpressure at the point in time T₁.

3. Method according to claim 1 or 2, wherein discriminating whether the bioreactor container (1) is sufficiently tight is effected by means of a fluid quantity M, which has been supplied to the bioreactor container (1) after the point in time T₁ in order to keep the overpressure P₁ constant.

4. Method according to one of the preceding claims, wherein discriminating whether the bioreactor container (1) is sufficiently tight is effected by means of a detection of fluid materials, which have been supplied to the bioreactor container (1), outside or within the bioreactor container (1).

5. Method according to one of the preceding claims, further comprising the steps:
- filling the bioreactor container (1) with educts;
- carrying out a chemical or biochemical reaction in the bioreactor container (1);
- discharging the contents from the bioreactor container (1).

6. Method according to one of the preceding claims, wherein discriminating whether the bioreactor container (1) is sufficiently tight is effected prior to and after carrying out the biochemical reaction in the bioreactor container (1).

7. Method according to one of the preceding claims, wherein the fluid-permeable reception layer comprises a woven textile, a non-woven textile and/or a foam.

8. Apparatus for testing the integrity of a bioreactor container comprising:
- a bioreactor container reception (25) for receiving a bioreactor container (1);
- at least one fluid-permeable and/or structured reception layer (7), which is replaceably arranged at least partially at the bioreactor container reception (25), wherein the reception layer (7) is construed to be arranged, at least in some areas, between a wall (3) of the bioreactor container (1) and the bioreactor container reception (25),
and wherein the structured reception layer has recesses having a depth of at least approximately 200 µm and/or wherein the structured reception layer has elevations having a width of at most approximately 200 µm; and
- a testing device (31) by which it can be discriminated whether the bioreactor container (1) is sufficiently tight,
wherein discriminating is effected by means of a pressure difference (P₂-P₁) between an overpressure P₂ at a later point in time T₂ and the predetermined overpressure at the point in time T₁,
and/or
by means of a fluid quantity M, which has been supplied to the bioreactor container (1) after the point in time T₁ in order to keep the overpressure P₁ constant.

9. Apparatus according to claim 8, further comprising:
- a fluid source (27), which is fluidically connectable to a container opening (9a, 9b, 9c, 9d) of the bioreactor container (1).

10. Apparatus according to claim 8 or 9,
wherein discriminating further is effected by means of a detection of fluid materials, which have been supplied to the bioreactor container (1), outside the bioreactor container (1).

11. Apparatus according to one of claims 8 to 10, wherein the fluid-permeable reception layer comprises a woven textile, a non-woven textile and/or a foam.

12. Use of a fluid-permeable and/or structured reception layer (7) for replaceable arrangement at a bioreactor container reception (25) of an apparatus for testing the integrity of a bioreactor container according to one of claims 8 to 11, wherein, after the arrangement of a bioreactor container (1) in the bioreactor container reception (25), the reception layer (7) at least in some areas is clamped in place by the bioreactor container and the bioreactor container reception (25) therebetween.

## Revendications

1. Procédé de contrôle de l'intégrité d'un récipient de bioréacteur (1) avec les étapes :
- mise à disposition d'un récipient de bioréacteur (1) avec une paroi étanche au fluide, flexible au moins par région (3) et avec au moins une ouverture de récipient (9a, 9b, 9c, 9d) ;
- mise à disposition d'un dispositif de contrôle avec un logement de récipient de bioréacteur (25) et une couche de logement perméable au fluide et/ou structurée (7) qui est disposée de manière permutable sur le logement de récipient de bioréacteur (25), dans lequel la couche de logement structurée présente des renfoncements qui ont au moins environ 200 µm de profondeur et/ou dans lequel la couche de logement structurée présente des élévations qui ont environ 200 µm de largeur maximum ;
- disposition du récipient de bioréacteur (1) au moins partiellement dans le logement de récipient de bioréacteur (25), dans lequel le récipient de bioréacteur (1) vient en contact avec sa paroi (3) au moins par région avec la couche de logement (7) dans le logement de récipient de bioréacteur (25);
- liaison de l'au moins une ouverture de récipient (9a, 9b, 9c, 9d) du récipient de bioréacteur à une source de fluide (27) ;
- remplissage du récipient de bioréacteur (1) d'un fluide provenant de la source de fluide (27) pour générer à un premier instant T₁ une surpression prédéterminée P₁ dans le récipient de bioréacteur (1) ;
- décider si le récipient de bioréacteur (1) est suffisamment étanche.

2. Procédé selon la revendication 1, dans lequel le fait de décider si le récipient de bioréacteur (1) est suffisamment étanche s'effectue à l'aide d'une différence de pression (P₂-P₁) entre une surpression P₂ à un instant ultérieur T₂ et la surpression déterminée à l'instant T₁.

3. Procédé selon la revendication 1 ou 2, dans lequel le fait de décider si le récipient de bioréacteur (1) est suffisamment étanche s'effectue à l'aide d'une quantité de fluide M qui a été amenée au récipient de bioréacteur (1) après l'instant T₁ pour maintenir la surpression P₁ constante.

4. Procédé selon une des revendications précédentes, dans lequel le fait de décider si le récipient de bioréacteur (1) est suffisamment étanche s'effectue à l'aide d'une détection de substances fluides qui ont été amenées au récipient de bioréacteur (1) en dehors ou au sein du récipient de bioréacteur (1).

5. Procédé selon une des revendications précédentes, comprenant en outre les étapes :
- remplissage du récipient de bioréacteur (1) d'éduits ;
- réalisation d'une réaction chimique ou biochimique dans le récipient de bioréacteur (1) ;
- évacuation du contenu du récipient de bioréacteur (1).

6. Procédé selon une des revendications précédentes, dans lequel le fait de décider si le récipient de bioréacteur (1) est suffisamment étanche s'effectue avant et après la réalisation de la réaction biochimique dans le récipient de bioréacteur (1).

7. Procédé selon une des revendications précédentes, dans lequel la couche de logement perméable au fluide comprend un textile tissé, un textile non tissé et/ou une mousse.

8. Dispositif de contrôle de l'intégrité d'un récipient de bioréacteur comprenant :
- un logement de récipient de bioréacteur (25) pour le logement d'un récipient de bioréacteur (1);
- au moins une couche de logement perméable au fluide et/ou structurée (7) qui est disposée de manière permutable au moins partiellement sur le logement de récipient de bioréacteur (25), dans lequel la couche de logement (7) est conçue pour être disposée au moins par région entre une paroi (3) du récipient de bioréacteur (1) et le logement de récipient de bioréacteur (25), et dans lequel la couche de logement structurée présente des renfoncements qui ont au moins environ 200 µm de profondeur et/ou dans lequel la couche de logement structurée présente des élévations qui ont environ 200 µm de largeur maximum ; et
- un dispositif de contrôle (31) par lequel il peut être décidé si le récipient de bioréacteur (1) est suffisamment étanche,
dans lequel le fait de décider s'effectue à l'aide d'une différence de pression (P₂-P₁) entre une surpression P₂ à un instant ultérieur T₂ et la surpression déterminée à l'instant T₁
et/ou
à l'aide d'une quantité de fluide M qui a été amenée au récipient de bioréacteur (1) après l'instant T₁ pour maintenir la surpression P₁ constante.

9. Dispositif selon la revendication 8, comprenant en outre :
- une source de fluide (27) qui peut être connectée de manière fluidique à une ouverture de récipient (9a, 9b, 9c, 9d) du récipient de bioréacteur (1).

10. Dispositif selon la revendication 8 ou 9,
dans lequel le fait de décider s'effectue en outre à l'aide d'une détection de substances fluides qui ont été amenées au récipient de bioréacteur (1) en dehors du récipient de bioréacteur (1).

11. Dispositif selon une des revendications 8 à 10, dans lequel la couche de logement perméable au fluide comprend un textile tissé, un textile non tissé et/ou une mousse.

12. Utilisation d'une couche de logement perméable au fluide et/ou structurée (7) pour la disposition permutable sur un logement de récipient de bioréacteur (25) d'un dispositif de contrôle de l'intégrité d'un récipient de bioréacteur selon une des revendications 8 à 11, dans laquelle la couche de logement (7) est coincée après la disposition d'un récipient de bioréacteur (1) dans le logement de récipient de bioréacteur (25) au moins par région par le récipient de bioréacteur (1) et le logement de récipient de bioréacteur (25) entre ceux-ci.
